# EUROPEAN PATENT APPLICATION

(11) **EP 4 620 312 A1**
(43) Date of publication of application: **24.09.2025**
(21) Application number: 23906715.0
(22) Date of filing: 05.12.2023
(51) Int. Cl.: A23L 33/125, A61K 31/7016, A61P 9/00, A61P 43/00

(54) **ENDOTHELIN-1 SECRETION INHIBITOR**

(30) Priority: 21.12.2022 JP 2022204344
(71) Applicant: Mitsui DM Sugar Co., Ltd., Tokyo 108-0014 (JP)
(72) Inventor: KOBAYASHI, Ryota, Tokyo 120-0045 (JP); SAKAZAKI, Miki, Tokyo 161-0034 (JP); NAGAI, Yukie, Tokyo 161-0034 (JP)
(74) Representative: Grünecker Patent- und Rechtsanwälte PartG mbB
(86) International application number: PCT/JP2023/043517
(87) International publication number: WO 2024/135349

(57) **Abstract**

The present invention relates to an endothelin-1 secretion inhibitor containing isomaltulose as an active ingredient.

## Description

### Technical Field

The present invention relates to an endothelin-1 secretion inhibitor.

### Background Art

Isomaltulose is a compound also referred to as 6-O-α-D-glucopyranosyl-D-fructose (palatinose as a trademark) and is known as a carbohydrate having various functionalities. For example, Patent Literature 1 describes that the deterioration of diabetic nephropathy is suppressed by isomaltulose ingestion.

### Citation List

### Patent Literature

Patent Literature 1: Japanese Unexamined Patent Publication No. 2016-41661

### Summary of Invention

### Technical Problem

An object of the present invention is to provide a novel use of isomaltulose for further effective use of isomaltulose.

### Solution to Problem

The present inventors have found that isomaltulose has an action of inhibiting secretion of endothelin-1, one of the peptide hormones, and have completed the present invention.

That is, one aspect of the present invention provides a secretion inhibitor of endothelin-1 containing isomaltulose as an active ingredient.

The endothelin-1 secretion inhibitor is preferably used such that isomaltulose is continuously administered to a human once or more a day for 4 weeks or more.

The endothelin-1 secretion inhibitor is preferably used such that isomaltulose is administered in an amount of 20 g or more per administration.

Abnormal production of endothelin-1 causes various diseases such as cancer, hypertension, and heart disease, but the endothelin-1 secretion inhibitor of the present invention inhibits the abnormal production of endothelin-1. It is also known that secretion of endothelin-1 is induced by postprandial hyperglycemia, but the endothelin-1 secretion inhibitor of the present invention can effectively inhibit the secretion of endothelin-1 even in a state where glucose is loaded, such as postprandial hyperglycemia.

### Advantageous Effects of Invention

According to the present invention, a novel use of isomaltulose is provided.

### Brief Description of Drawings

FIG. 1 is a graph showing a transition of the concentration of endothelin-1 in a test according to an example.
FIG. 2 is a result of statistical analysis on a transition of the concentration of endothelin-1 in a test according to an example.

### Description of Embodiments

Hereinafter, embodiments of the present invention will be described. However, the present invention is not limited to the following embodiments.

The endothelin-1 secretion inhibitor according to the present embodiment contains isomaltulose as an active ingredient.

Endothelin-1, a peptide hormone composed of 21 amino acid residues discovered in 1988, is one of potent vasoconstrictors in a living body. Although endothelin-1 has vascular smooth muscle contraction activity derived from vascular endothelium, the endothelin-1 is produced not only in vascular endothelium but also in cells such as cardiomyocytes and renal glomerular mesangial cells. Endothelin-1 transmits a signal through a cell membrane by activating G proteins such as Gi protein and Gq protein in a cell through an endothelin receptor. **Endothelin-1** apparently binds irreversibly to the endothelin receptor, regulates blood flow, and contributes to sustained increases in blood pressure.

Besides blood flow control, endothelin-1 plays a role in various actions such as development of nerve cells, proliferation of cells, adjustment of water concentration in body fluid, positive chronotropic and inotropic action, vascular smooth muscle proliferation, cardiomyocyte hypertrophy, and fibroblast proliferation. Therefore, the endothelin-1 secretion inhibitor according to the present embodiment can also exert an effect of controlling these actions through inhibiting the secretion of endothelin-1.

Isomaltulose is a compound formed by α-1,6 bonding of glucose and fructose, and is also referred to as 6-O-α-D-glucopyranosyl-D-fructose. Isomaltulose is also referred to as palatinose. Note that "PALATINOSE" is a registered trademark of Mitsui DM Sugar Co., Ltd.

Isomaltulose is found naturally in honey. It is also present in a transfer product generated when an α-glucosyltransferase (isomaltulose synthase) derived from bacteria or yeast acts on sucrose. Industrially, isomaltulose is produced by causing an α-glucosyltransferase derived from bacteria such as *Protaminobacter rubrum* and *Serratia plymuthica* to act on sucrose.

The isomaltulose used may be naturally derived or synthesized by an enzymatic action or the like.

Isomaltulose may be contained in the endothelin-1 secretion inhibitor as crystal particles or may be contained as granular particles. The granular particles may be, for example, (spherical) particles containing an aggregate of a plurality of crystal particles of isomaltulose and an amorphous sugar content, and the sugar content is contained in the aggregate of crystal particles. Such granular particles can be obtained, for example, by a method of precipitating crystal particles of isomaltulose from a sugar solution containing isomaltulose and an amorphous sugar content and spray-drying the sugar solution containing the crystal particles. Alternatively, granular particles containing isomaltulose can be obtained by a method of applying a shear force to the sugar solution while heating the sugar solution to precipitate crystal nuclei of isomaltulose and then cooling a mixture containing the crystal nuclei. The sugar solution can be obtained, for example, by causing an enzyme to act on sucrose. In this case, the sugar solution and the granular particles obtained contain trehalose, fructose, glucose, sucrose, isomaltose, and the like as amorphous sugar contents. The granular particles may be solid matters described in Japanese Unexamined Patent Publication No. 2012-179045 or the like.

In addition, commercially available isomaltulose may be used. Examples of such a commercially available product include crystalline palatinose (trade name: "Crystalline Palatinose PST-N", manufactured by Mitsui DM Sugar Co., Ltd.), powder palatinose (trade name: "Powder Palatinose PST-NP", manufactured by Mitsui DM Sugar Co., Ltd.), and palatinose syrup (trade names: "Palatinose Syrup-ISN" and "Palatinose Syrup-TN", manufactured by Mitsui DM Sugar Co., Ltd.).

The endothelin-1 secretion inhibitor according to the present embodiment only needs to contain isomaltulose as an active ingredient, and may be composed of isomaltulose alone, or may be a composition containing isomaltulose. When the endothelin-1 secretion inhibitor is a composition, isomaltulose contained therein may be contained as isomaltulose alone or contained as a commercially available isomaltulose preparation containing several saccharides. The isomaltulose preparation may contain an additional component in addition to isomaltulose.

The additional component may be a material that can be used for foods, quasi-drugs, or pharmaceuticals. The material that can be used for foods, quasi-drugs, or pharmaceuticals is not particularly limited, and examples thereof include an amino acid, a protein, a carbohydrate, a fat or oil, a sweetener, a mineral, a vitamin, a fragrance, an excipient, a binder, a lubricant, a disintegrant, an emulsifier, a surfactant, a base, a solubilizing agent, and a suspending agent.

Examples of the protein include milk casein, whey, soybean protein, wheat protein, and albumen. Examples of the carbohydrate include corn starch, cellulose, pregelatinized starch, wheat starch, rice starch, and potato starch. Examples of the oil include salad oil, corn oil, soybean oil, safflower oil, olive oil, and palm oil. Examples of the sweetener include saccharides such as glucose, sucrose, fructose, glucose-fructose syrup, and fructose-glucose syrup, sugar alcohols such as xylitol, erythritol, and maltitol, artificial sweeteners such as sucralose, aspartame, saccharin, and acesulfame K, and stevia sweeteners. Examples of the mineral include calcium, potassium, phosphorus, sodium, manganese, iron, zinc, magnesium, and salts thereof. Examples of the vitamin include vitamin E, vitamin C, vitamin A, vitamin D, vitamin B, biotin, and niacin. Examples of the excipient include dextrin, starch, lactose, and crystalline cellulose. Examples of the binder include polyvinyl alcohol, gelatin, hydroxypropyl methyl cellulose, hydroxypropyl cellulose, sodium carboxymethyl cellulose, and polyvinylpyrrolidone. Examples of the lubricant include magnesium stearate, calcium stearate, and talc. Examples of the disintegrant include crystalline cellulose, agar, gelatin, calcium carbonate, sodium hydrogen carbonate, and dextrin. Examples of the emulsifier or surfactant include sucrose fatty acid ester, citric acid, lactic acid, glycerin fatty acid ester, polyglycerin fatty acid ester, sorbitan fatty acid ester, propylene glycol fatty acid ester, and lecithin. Examples of the base include cetostearyl alcohol, lanolin, and polyethylene glycol. Examples of the solubilizing agent include polyethylene glycol, propylene glycol, sodium carbonate, and sodium citrate. Examples of the suspending agent include glyceryl monostearate, polyvinyl alcohol, polyvinyl pyrrolidone, methyl cellulose, hydroxymethyl cellulose, and sodium alginate. These may be used singly or in combination with two or more thereof.

Since isomaltulose has a sweetness of about 1/2 of sucrose, the endothelin-1 secretion inhibitor according to the present embodiment may mainly contain isomaltulose as a sweetener. Since the isomaltulose has a low hydrolysis rate of 1/5 of sucrose and is capable of inhibiting an increase in blood glucose level after ingestion, inclusion of isomaltulose mainly as a sweetener also produces an effect of facilitating blood glucose control.

When the endothelin-1 secretion inhibitor contains a component other than isomaltulose, the content of isomaltulose may be appropriately set according to the form, intended use, and the like of the endothelin-1 secretion inhibitor, but for example, may be 10 mass% or more, 20 mass% or more, or 30 mass% or more, and 70 mass% or less, 60 mass% or less, or 50 mass% or less with respect to the total amount of the endothelin-1 secretion inhibitor.

The endothelin-1 secretion inhibitor can be used as a food composition or a pharmaceutical composition. The food composition or pharmaceutical composition according to the present embodiment may be provided in the form of, for example, a health food, a food for specified health uses, a functional food, a nutraceutical food, a supplement, a quasi-drug, or a pharmaceutical. The food composition according to the present embodiment may be labeled, for example, to inhibit the secretion of endothelin-1 and to inhibit the secretion of over-secreted endothelin-1. In addition, the form of the inhibitor may be any form such as a solid (powder, granules, etc.), a liquid (solution, suspension, etc.), or a paste and may be any dosage form such as a powder, a pill, a granule, a tablet, a capsule, a troche, a liquid, or a suspension.

The endothelin-1 secretion inhibitor may be administered once or more a day. The endothelin-1 secretion inhibitor may be administered twice or more, or 3 times or more a day. The endothelin-1 secretion inhibitor may be administered up to 3 times a day.

The time of day when the endothelin-1 secretion inhibitor is administered is not particularly limited, but may be, for example, on an empty stomach before breakfast, simultaneously with breakfast, before lunch, or simultaneously with lunch.

The endothelin-1 secretion inhibitor may be continuously administered for 4 weeks or more from the viewpoint of more effectively inhibiting the secretion of endothelin-1. The endothelin-1 secretion inhibitor may be administered for 8 weeks or more, or 12 weeks or more. The endothelin-1 secretion inhibitor may be administered for 52 weeks or less.

The endothelin-1 secretion inhibitor is preferably used such that isomaltulose is administered in an amount of preferably 20 g or more, more preferably 22 g or more, and still more preferably 25 g or more per administration. In addition, the endothelin-1 secretion inhibitor may be used such that isomaltulose is administered in an amount of 75 g or less, 72 g or less, or 70 g or less per administration. The endothelin-1 secretion inhibitor is preferably used such that isomaltulose is administered in an amount of preferably 20 g or more, more preferably 22 g or more, and still more preferably 25 g or more per day. In addition, the endothelin-1 secretion inhibitor may be used such that isomaltulose is administered in an amount of 75 g or less, 72 g or less, or 70 g or less per day. Within this range, a sufficient blood concentration can be achieved, and the action of inhibiting the secretion of endothelin-1 can be exhibited more effectively.

The endothelin-1 secretion inhibitor may be administered orally or administered parenterally, such as intravenously. The endothelin-1 secretion inhibitor is preferably administered orally.

The subject to be administered with the endothelin-1 secretion inhibitor may be a human or an animal, and is preferably a human. The human as the subject to be administered may be a human (healthy person) not suffering from diabetes. The human as an administration subject may be an elderly person of 60 years old or more, 65 years old or more, or 70 years old or more.

When used for animals, the endothelin-1 secretion inhibitor may also be used as feed or feed additive. Examples of the feed include feed for companion animals such as dog food and cat food, feed for livestock, feed for poultry, and feed for cultured fish and shellfish. "Feed" includes anything orally ingested by an animal for nutritional purposes. More specifically, when classified in terms of nutrient content, all of roughage, concentrate feed, inorganic feed, and special feed are included, and when classified in terms of public standards, all of blended feed, mixed feed, and single feed are included. In addition, when classified in terms of a feeding method, all of feed to be directly fed, feed to be mixed with other feed and fed, or feed to be added to drinking water to supplement nutrients are included.

One embodiment of the present invention may also be regarded as a method for inhibiting secretion of endothelin-1, the method including the step of administering an effective amount of an endothelin-1 secretion inhibitor containing isomaltulose as an active ingredient to a subject in need thereof. The subject in the above method may be a human or an animal, and is preferably a human. Aspects, administration methods, dosages, and the like of the endothelin-1 secretion inhibitor may be the same as those described above. In addition, one embodiment of the present invention may be regarded as isomaltulose for use in a method for inhibiting secretion of endothelin-1.

One embodiment of the present invention may also be regarded as the use of isomaltulose in the production of an endothelin-1 secretion inhibitor. Aspects of the endothelin-1 secretion inhibitor may be the same as those described above. One embodiment of the present invention may also be regarded as the use of isomaltulose in inhibiting secretion of endothelin-1.

### Examples

Hereinafter, the present invention will be described in more detail based on Examples. However, the present invention is not limited to the following Examples.

According to the following method, the influence of isomaltulose ingestion on the secretion of endothelin-1 was examined.

### <Subject>

Subjects were 54 healthy middle to elderly persons (30 males and 24 females). All subjects were assigned to one of the groups, i.e., an isomaltulose ingestion group (Group I) and a sucrose ingestion group (Group S), in a single blind test before intervention, and the intervention was performed for 12 weeks each.

### <Test Design>

All subjects ingested jellies having compositions described in Table 1 once a day for 12 weeks continuously. Group I received a jelly containing isomaltulose (Example 1), and group S received a jelly containing sucrose (Comparative Example 1). The jelly was taken every morning before breakfast, and all subjects were instructed not to change their dietary habits during the intervention period from those before the intervention. A 75 g oral glucose tolerance test (75-g OGTT, described in detail below) was performed before the 12-week intervention and at 4 weeks, 8 weeks, and 12 weeks after the start of the intervention, after the lapse of 1 day or more from the ingestion of the jelly and the secretion amount of endothelin-1 was evaluated by the method described below before the 75-g OGTT and at 60 minutes and 120 minutes after the 75-g OGTT.

**[Table 1]**

| Jelly composition (g) | Comparative Example 1 | Example 1 |
|---|---|---|
| Gelling agent | 0.7 | 0.7 |
| Sucrose | 25 | - |
| Isomaltulose | - | 25 |
| Acidulant (50% citric acid) | 0.2 | 0.2 |
| Fragrance | 0.2 | 0.2 |
| Defoamer | 0.03 | 0.03 |
| Water | 73.87 | 73.87 |
| Total | 100 | 100 |

### <75 g Oral Glucose Tolerance Test (75-g OGTT)>

The 75-g OGTT was performed under the instruction of a doctor using TRELAN- G 75 g (Yoshindo Inc.), which is generally used in medical institutions and research, and the test solution was orally ingested in a general volume (225 mL per dose) within 5 minutes according to the guidelines of the Japan Diabetes Society (Araki E, Goto A, Kondo T, Noda M, Noto H, Origasa H, et al. Japanese Clinical Practice Guideline for Diabetes 2019. J Diabetes Investig 2020; 11: 1020-1076.).

### <Evaluation of Secretion Amount of Endothelin-1>

For all subjects, blood was collected from the cubital vein in a fasting state for 12 hours or more in a hygienic environment. Before ingestion of a carbohydrate solution for the 75-g OGTT and at 60 minutes and 120 minutes after the ingestion, a nurse collected blood (15 to 20 mL per collection) using a sterilized disposable blood collection needle under the instruction of a doctor. A sample after blood collection was immediately centrifuged at 4°C and 3000 rpm for 15 minutes, and the concentration of endothelin-1 was measured by ELISA (enzyme-linked immunosorbent assay) using a test kit (Cayman Chemical, Ann Arbor) for measuring the concentration of endothelin-1.

### <Statistical Processing>

A change in endothelin-1 concentration before and after the start of the intervention of each group was shown as the average value (95% confidence interval). For the comparison of the change in endothelin-1 concentration between the 2 groups before and after the 75-g OGTT, a two-way repeated measures ANOVA was used. The Bonferroni method was performed for comparison of changes in each intervention in post-hoc test. The total area under the curve at 90 minutes (endothelin-1 concentration AUC) was calculated using the trapezoidal rule. For statistical analysis, IBM SPSS Statistics Ver. 25 (manufactured by IBM Corporation) was used. The statistical significance level in this test was set at 5%.

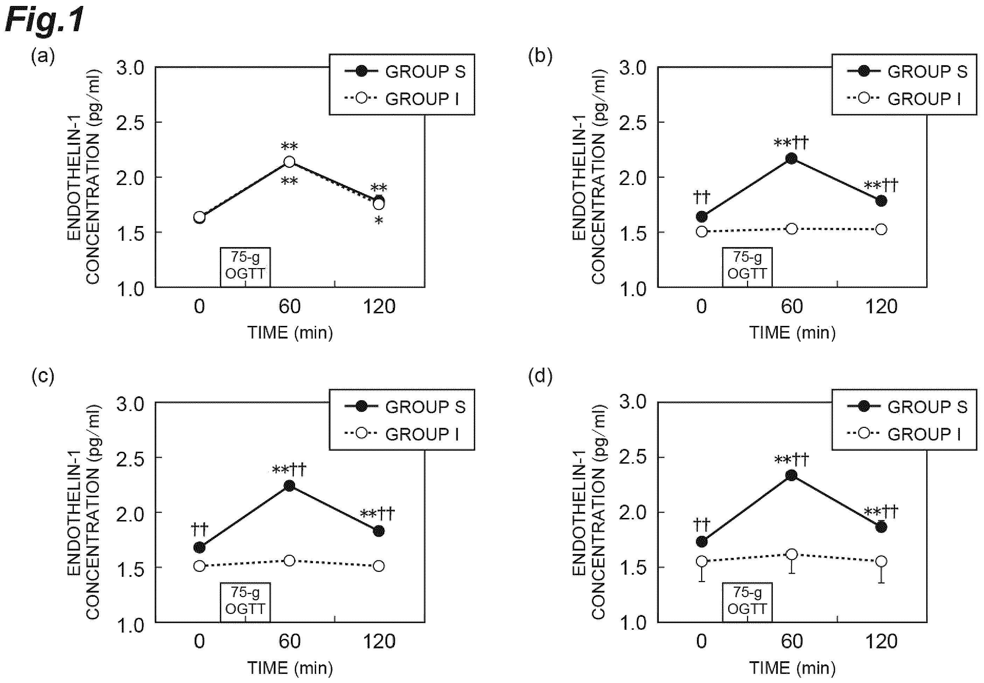

FIG. 1 shows the transition of the endothelin-1 concentration of each group before and after the start of the intervention. FIG. 1(a) shows the transition of the endothelin-1 concentration before the intervention and shows the transition of the endothelin-1 concentration before the 75-g OGTT, at 60 minutes after the 75-g OGTT, and at 120 minutes after the 75-g OGTT. FIGS. 1(b), (c), and (d) show the endothelin-1 concentration at 4, 8, and 12 weeks after the start of the intervention, respectively, and show the transition of the endothelin-1 concentration before the 75-g OGTT, at 60 minutes after the 75-g OGTT, and at 120 minutes after the 75-g OGTT.

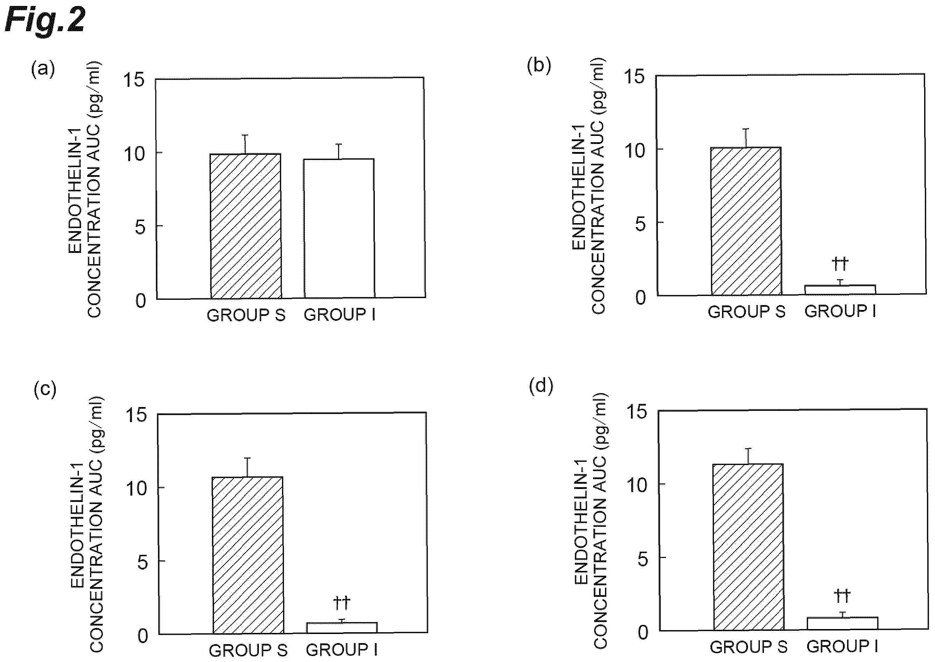

FIG. 2 shows the endothelin-1 concentration AUC for each group before and after the start of the intervention. FIGS. 1(a), (b), (c), and (d) show the endothelin-1 concentration AUC for each group before the intervention, and at 4, 8, and 12 weeks after the start of the intervention, respectively.

The endothelin-1 concentration before the intervention significantly increased in both groups at 60 minutes (p < 0.05) and 120 minutes (p < 0.05) after the 75-g OGTT compared to before the 75-g OGTT, and no significant difference was found between both groups. The endothelin-1 concentration at 4, 8, and 12 weeks after the start of the intervention significantly increased in group S at 60 minutes (p < 0.01) and 120 minutes (p < 0.01) after the 75-g OGTT compared to before the 75-g OGTT, but no significant change was found in Group I. The endothelin-1 concentration before the 75-g OGTT and at 60 minutes **and 120** minutes after the 75-g OGTT showed a significantly lower value at 4, 8, and 12 weeks after the start of the intervention in Group I than in Group S (p < 0.01). No group difference was observed in the endothelin-1 concentration AUC before the intervention. The endothelin-1 concentration AUC at 4, 8, and 12 weeks after the start of the intervention showed a significantly lower value in Group I than in Group S (p < 0.01). As described above, the endothelin-1 concentration at 60 minutes and 120 minutes after the 75-g OGTT was lower in Group I than in Group S at 4, 8, and 12 weeks after the start of the intervention, indicating that that the ingestion of isomaltulose inhibited the secretion of endothelin-1.

## Claims

1. An endothelin-1 secretion inhibitor comprising:
isomaltulose as an active ingredient.

2. The endothelin-1 secretion inhibitor according to claim 1, wherein the endothelin-1 secretion inhibitor is used such that the isomaltulose is continuously administered to a human once or more a day for 4 weeks or more.

3. The endothelin-1 secretion inhibitor according to claim 1 or 2, wherein the endothelin-1 secretion inhibitor is used such that the isomaltulose is administered in an amount of 20 g or more per administration.
